# DEMANDE DE BREVET EUROPEEN

(11) **EP 1 020 264 A1**
(43) Date de publication de la demande: **19.07.2000**
(21) Numéro de dépôt: 98124852.9
(22) Date de dépôt: 31.12.1998
(51) Int. Cl.: B27K 5/00, B27K 5/06

(54) **Composition enzymatique et procédé de séchage du bois**

(71) Demandeur: N.V.ETS ROBERT STIERNON S.A., 7850 Petit-Enghien (BE)
(72) Inventeur: Henry, Olivier, 1300 Wavre (BE)
(74) Mandataire: Vandeberg, Marie-Paule L.G.

(57) **Abrégé**

L'invention concerne une composition enzymatique pour le séchage du bois ainsi qu'un procédé rapide de séchage du bois destiné à des applications alimentaires. Le procédé consiste, dans un premier temps, à mettre le bois frais en contact avec une solution enzymatique et dans un deuxième temps à effectuer un séchage classique en étuve.

L'invention permet d'améliorer les caractéristiques d'imprégnabilité des bois ainsi que d'améliorer les qualités aromatiques et olfactives des bois.

## Description

La présente invention concerne une composition enzymatique pour le séchage du bois, une solution aqueuse pour le traitement du bois, ainsi qu'un procédé de séchage ou de maturation du bois utilisant cette composition enzymatique ou cette solution enzymatique.

### ETAT DE LA TECHNIQUE :

L'utilisation de bois comme matériau en construction, menuiserie, ébénisterie ou tonnellerie suppose un séchage préalable afin de stabiliser celui-ci dimensionnellement.

Pour la plupart des applications industrielles, ce séchage se fait en étuves à températures et humidités contrôlées.

Pour d'autres applications notamment dans les cas où le bois sera par la suite en contact avec des aliments, le séchage se fait naturellement, à l'extérieur pendant des durées assez longues, dans le but d'éliminer certains composés indésirables. La durée de ce séchage naturel ou vieillissement est variable en fonction de l'utilisation finale. Elle est généralement de l'ordre de 2 ans.

Dans le cas de séchage naturel, le bois est colonisé par une flore fongique qui peut transformer certains composés du bois. Ces transformations du bois ne sont cependant pas contrôlables. Les bois séchés naturellement présentent une grande variabilité de qualité.

Par contre, avec un séchage artificiel en étuve, ces transformations n'ont pas lieu et le bois ainsi séché n'est pas approprié pour des applications alimentaires car il ne permet pas d'éliminer les molécules indésirables (telles que des molécules toxiques ou amères).

### DESCRIPTION DE L'INVENTION

L'invention a pour but de fournir un procédé rapide de séchage du bois qui puisse être approprié pour des applications alimentaires.

La présente invention a pour objet une composition enzymatique pour le séchage du bois comprenant principalement des enzymes de la classe des hydrolases, ainsi qu'une solution aqueuse pour le séchage du bois comprenant de 0.01% à 5% et de préférence de 0.1 à 0.5% d'enzymes de la classe des hydrolases par rapport au volume de bois à traiter.

La présente invention a également pour objet un procédé de séchage du bois comportant une étape de mise en contact du bois avec une solution enzymatique et, de préférence, une étape ultérieure de séchage en étuve.

Des modes de réalisations particuliers de l'invention sont décrits dans les revendications dépendantes.

La composition enzymatique utilisée peut comprendre des enzymes de type exocellulaires ou endocellulaires. Les enzymes peuvent être d'origine bactérienne, fongique ou de toute autre origine possible.

### DESCRIPTION DETAILLEE DE L'INVENTION

Le bois à traiter est plongé dans un bain contenant de l'eau ou tout autre solvant approprié et des quantités variables d'enzymes de la classe des hydrolases (classe 3 de l'union international de biochimie (IUB)) de préférence choisies parmi les enzymes de type glucosidase (sous-classe 3.2 de la classification IUB), et/ou les enzymes de type lipases (sous-classe 3.1. de cette même classification), etc., et les mélanges de ces enzymes. Les enzymes de la sous-classe (3.2.) sont généralement connues sous le nom de cellulases. Parmi les glucosidases utilisables pour la présente invention, on peut notamment citer les cellobiohydrolases, les endoglucanases, les β-glucosidases, les hemicellulases, les amylases, et les xylanases, etc.

Après ce traitement par immersion pouvant durer de 1h à 1 semaine en fonction de l'intensité de l'effet désiré, du mélange d'enzymes utilisé, du volume à traiter, du volume du bain, de la nature du bois traité et de la température et du pH du bain, le bois est alors mis en étuve à température et humidité contrôlées pour réaliser un séchage classique.

Les études microscopiques réalisées sur du bois traité suivant l'invention ont montré que le traitement modifie de façon superficielle l'ultrastructure du bois En particulier, on a observé que le procédé de séchage suivant l'invention permet, généralement sur 1 à 4 mm de profondeur, l'ouverture des pores normalement bouchés lors d'un séchage artificiel classique ainsi qu'une diminution de l'épaisseur des parois cellulaires du bois. Ces modifications permettent avantageusement de faciliter l'évacuation de l'eau lors du passage ultérieur en étuve.

L'ouverture de ces pores, aussi appelés ponctuations, permettra avantageusement par la suite une imprégnation plus complète du bois par des produits phytosanitaires de traitement et/ou de préservation par exemple.

Il s'est avéré que le procédé suivant l'invention permet une hydrolyse des ellagitannins solubles, naturellement présent dans le bois, d'une partie des ellagitannins liés aux polysaccharides pariétaux, ainsi que de diverses molécules aromatiques qui sont transformées, par le procédé suivant l'invention, en molécules gustativement neutres (telles que l'acide digallique, ou les coumarines hétérosidiques).

Le procédé suivant l'invention permet donc d'améliorer l'impression gustative des extraits de bois en éliminant une partie significative des composés indésirables comme les composés phénoliques et en hydrolysant des formes jugées amères. Ceci est particulièrement important pour les bois devant par la suite entrer en contact avec des aliments (typiquement bois de tonnellerie)

La présente invention a encore comme avantage de fournir un procédé rapide de vieillissement du bois tout en respectant l'ensemble des réactions physicochimiques liées au séchage naturel du bois.

De plus, l'invention a l'avantage de fournir un procédé reproductible de séchage du bois et de permettre une meilleure homogénéité de la qualité des bois traités entre des espèces de bois différentes ou de provenance différente ou entre les différentes parties d'un même arbre. On retrouve donc une meilleure constance dans les propriétés physico-chimiques (mécaniques et aromatiques notamment) du bois traité selon l'invention par rapport au bois séché de manière naturelle.

Le procédé suivant l'invention permet également par un choix approprié d'enzymes présentant des activités particulières, de conférer au bois des propriétés particulières telles que l'expression d'arômes ou odeurs particuliers

### EXEMPLE PARTICULIER DE REALISATION :

Dans une cuve de 15.000 litres d'eau, on immerge 8 piles d'environ 1 m³ de douelles de chêne superposées en quinconce, un espace étant ménagé entre chaque douelle pour permettre la circulation de la solution. La température de l'eau est ajustée à 40 C° et le bois est laissé à tremper pendant 24 heures.

Ensuite, on ajoute 8 kg d'une formulation enzymatique comportant principalement :
- des cellulases (n° IUB EC 3.2.1.4) ayant
   - une activité mesurée en unité endocellulase de 10.000 ECU/g de formulation,
   - une activité mesurée en unité xylanase de 6.000 BXU/g de formulation, et
   - une activité mesurée en unité mannanase de 4.000 MNU/g de formulation,
- des α-amylases (n°IUB EC 3.2.1.1) à une activité mesurée en unité 〈〈α thermo-stable〉〉 de 10.000 αTU/g de formulation.

Ces concentrations sont bien entendu données à titre d'exemple. Il est clair qu'elles pourront varier considérablement en fonction des différents paramètres utilisés pour le traitement (temps de traitement, intensité désirée, nature du bois, volume du bain, volume de bois à traiter, pH et température du bain, etc.)

On ajuste ensuite le pH du bain à environ 4,5 - 5,0. On laisse agir la solution enzymatique en maintenant une faible circulation afin d'assurer une répartition homogène des enzymes dans le bain.

Les bois sont ainsi traités pendant 48 heures puis sortis du bain et passés en étuve pendant deux semaines pour leur permettre d'atteindre une humidité de 18 % environ. Les douelles sont alors prêtes pour la fabrication de tonneaux, par exemple.

Les bois ainsi traités ont montré un enrichissement aromatique très intéressant. En particulier, la teneur en Eugenol, mesurée suivant la méthode ci-dessous, a doublé par rapport aux bois séchés de manière naturelle et la teneur en Vanilline a augmenté de 50 %.

### Méthode d'extraction et d'analyse des composés volatils

### Première étape de l'extraction

1. Peser environ 5 lamelles de bois issues d'une douelle telle que traitée dans l'exemple ci-dessus.
2. Ajouter 150 ml d'une solution acétone/H₂O déminéralisée dans un rapport 7/3 Vol/Vol (= solution 1) et couvrir avec un film de paraffine.
3. Agiter pendant environ 48 heures à température ambiante.

### Deuxième étape de l'extraction

1. Evaporer l'acétone à l'aide d'un évaporateur rotatif à 30°C.
2. Recueillir la partie aqueuse dans un récipient fermé d'environ 100 ml.
3. Y ajouter d'abord 4 ml d'une solution d'hexane/diéthyléther 1/1 Vol/Vol (solution 2)
4. Agiter 5 minutes à vitesse moyenne.
5. Transvaser le mélange dans une ampoule à décanter et recueillir la phase organique.
6. Répéter cette opération à deux reprises en ajoutant cette fois 2 ml de la solution 2.
7. Les trois phases organiques sont rassemblées pour un même échantillon (environ 8 ml de fraction).
8. L'extrait est ensuite analysé par chromatographie en phase gazeuse dans une troisième étape avec un instrument du type GCMS.

## Revendications

1. Composition enzymatique pour le séchage du bois comprenant des enzymes de la classe des hydrolases.

2. Composition suivant la revendication précédente, caractérisé en ce que les enzymes sont choisies parmi les glucosidases, les cellulases, les amylases, les xylanases, les peroxydases, les lipases et les mélanges de ces enzymes.

3. Composition suivant la revendication 2, caractérisée en ce que les enzymes de type cellulase sont choisies parmi les cellobiohydrolases, les endoglucanases, les B-glucosidases, les hemicellulases.

4. Composition suivant l'une quelconque des revendications précédentes pour améliorer l'imprégnabilité du bois.

5. Composition suivant l'une quelconque des revendications précédentes pour la modification des caractéristiques aromatiques des bois.

6. Composition suivant l'une quelconque des revendications précédentes pour la modification des caractéristiques olfactives des bois.

7. Solution aqueuse pour le traitement du bois comportant de 0.01 à 5% d'enzymes de la classe des hydrolases par rapport au volume de bois à traiter.

8. Procédé de séchage du bois caractérisé en ce qu'il comporte une étape préalable de mise en contact du bois avec une solution de la composition enzymatique suivant l'une quelconque des revendications 1 à 6 ou avec une solution selon la revendication 7.

9. Procédé suivant la revendication précédente, caractérisé en ce qu'il comporte une étape ultérieure de séchage en étuve.

10. Procédé suivant l'une quelconque des revendications 8 et 9, caractérisé en ce que la mise en contact du bois avec la solution est effectuée par immersion du bois durant une période variant de 30 minutes à 2 semaine.

11. Procédé suivant l'une quelconque des revendications 8 à 10, caractérisé en ce que, de la mise en contact du bois avec ladite solution, il résulte une ouverture des pores du bois en moins de 2 semaines.
